(19) Europäisches Patentamt / European Patent Office / Office européen des brevets

(11) **EP 1 867 296 B1**

(12) **EUROPEAN PATENT SPECIFICATION**

(45) Date of publication and mention
of the grant of the patent:
**16.01.2019 Bulletin 2019/03**

(51) Int Cl.:
***A61B 18/14*** *(2006.01)*

(21) Application number: **07011488.9**

(22) Date of filing: **12.06.2007**

(54) **High frequency treatment instrument**

Hochfrequenzbehandlungsgerät

Instrument de traitement des hautes fréquences

(84) Designated Contracting States:
**DE FR GB IE**

(30) Priority: **14.06.2006 JP 2006164630**

(43) Date of publication of application:
**19.12.2007 Bulletin 2007/51**

(73) Proprietor: **Olympus Corporation**
**Tokyo 192-8507 (JP)**

(72) Inventors:
 • **Suzuki, Keita**
 **Hachioji-shi,**
 **Tokyo 192-8507 (JP)**

 • **Kimura, Megumi**
 **Hachioji-shi,**
 **Tokyo 192-8507 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**EP-A- 1 743 589     WO-A-01/15617**
**US-A- 4 615 330**

## Description

The present invention relates to a high frequency treatment instrument and a method of manufacturing such an instrument.

Description of the Related Art

Recently, in fields such as medial services, high frequency treatment instruments that perform various treatments while checking observation images taken by an endoscope are being used. One conventionally known high frequency treatment instrument dissects, solidifies, and stops bleeding of living tissue by supplying a high frequency current to treatment electrodes provided on a distal end thereof (for example see Japanese Unexamined Patent Application, First Publication No. 2005-58344). To efficiently supply the high frequency current from a high frequency power source to the treatment electrodes at this time, it is preferable to reduce impedance against the high frequency current as much as possible.

However in the conventional high frequency treatment instrument, high frequency current is supplied to forceps pieces, which constitutes the treatment electrodes, via an operation wire for opening and closing the forceps pieces. The operation wire therefore requires excellent mechanical characteristics such as tension and rotational tracking. That is, the mechanical characteristics are prioritized over current supply efficiency. Consequently, there is a limit on the reduction of the overall impedance against the high frequency current.

EP-A 1 743 589 describes a high frequency treatment instrument which aims already at decreasing the impedence against the high-frequency current to apply such current efficiently to a treatment portion. To this end, this prior art teaches a multiple-threaded coil connected electrically to the treatment portion. Document WO-A-01/15617 describes a high frequency treatment instrument according to the preamble of claim 1.

The present invention wishes to further reduce the impendence against the high-frequency current and to apply such current most efficiently to the treatment portion of the patient.

The high-treatment instrument according to the present invention to solve this problem is described in claim 1. The claim dependent from claim 1 describes a preferred embodiment of the instrument. Any embodiment which is in contradiction to the subject-matter of claim 1 is not part of the invention.

This high frequency treatment instrument of second aspect can adjust the flow of high frequency current to a current of a more appropriate magnitude by changing the impedance of the electrical circuit including the high frequency power.

In a third aspect of the high frequency treatment instrument according to the present invention is the high frequency treatment instrument in which the impedance adjust part changes the impedance of the electrical circuit when the treatment electrode contacts the target tissue.

This high frequency treatment instrument of third aspect changes the impedance of the electrical circuit when the treatment electrode actually contacts the target tissue, whereby a high frequency current of a more appropriate magnitude can be supplied at the time of the treatment.

In a fourth aspect of the high frequency treatment instrument according to the present invention is the high frequency treatment instrument in which the impedance adjust part includes a coil part having an inductance or a capacitor having a capacitance, and is connected in series with the electrical circuit.

According to this high frequency treatment instrument of fourth aspect, for example, when the inductance of the electrical circuit is large, the impedance adjust part can include the capacitor part, and when the capacitance of the electrical circuit is large, the impedance adjust part can include the coil part. In these cases, the square of the imaginary number part of the impedance of the electrical circuit can be changed to be minimized, and the magnitude of the impedance can be reduced.

In a fifth aspect of the high frequency treatment instrument according to the present invention is the high frequency treatment instrument in which the impedance adjust part includes an electrical resistor, and is connected in series with the electrical circuit.

This high frequency treatment instrument of fifth aspect can change the overall impedance by changing the actual number part of the impedance of the electrical circuit. Even if the load characteristics of the high frequency power source fluctuate due to fluctuation in the impedance of the electrical circuit that incorporates the impedance of the target tissue, the magnitude of the electrical resistor can be adjusted to obtain an output approximately at a rating power.

In a sixth aspect of the high frequency treatment instrument according to the present invention is the high frequency treatment instrument in which the treatment electrode includes an elongated coil sheath connected to a distal end thereof, and the coil sheath constitutes the coil part.

This high frequency treatment instrument of sixth aspect uses the coil sheath as an impedance adjust part which can adjust the inductance by changing the wire diameter, material, and winding number of the coil sheath.

In a seventh aspect of the high frequency treatment instrument according to the present invention is the high frequency treatment instrument in which the treatment electrode includes a first electrode and a second electrode that create mutually different potential differences.

This high frequency treatment instrument of seventh aspect can change the impedance of the electrical circuit incorporating the impedance between supply lines to the first electrode and the second electrode.

In an eighth aspect of the high frequency treatment instrument according to the present invention, an electrical circuit which is connected to a high frequency power source which generates a high frequency voltage in which at least one treatment electrode is included for performing conduction treatment using a high frequency current to a target tissue by applying a high frequency voltage from the

## BRIEF DESCRIPTION OF THE DRAWINGS

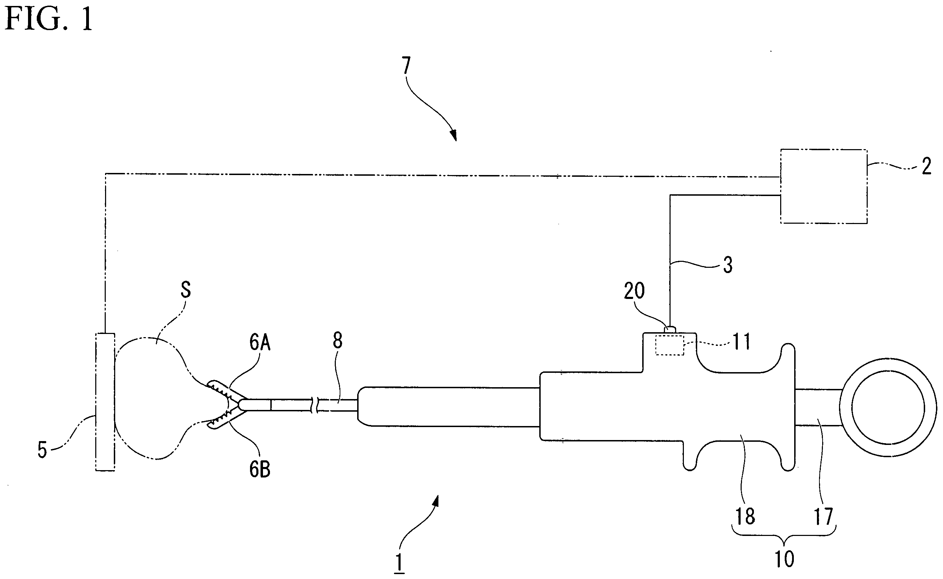

FIG. 1 is an overall summary view of a high frequency forceps instrument according to a first embodiment.

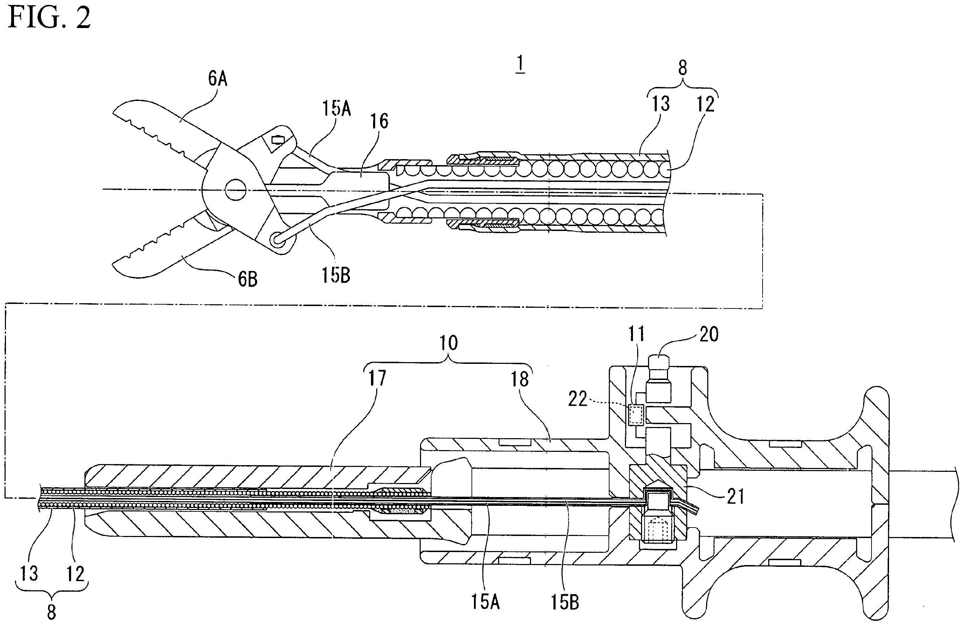

FIG. 2 is a side view of primary parts of a high frequency forceps instrument according to the first embodiment.

FIG. 3 is an overall summary view of a high frequency forceps instrument the second embodiment.

FIG. 4 is a partial cross-sectional plan view of primary parts of a papillotomy knife

FIG. 5 is a partial cross-sectional plan view of primary parts of a high frequency knife.

FIG. 6 is a partial cross-sectional plan view of primary parts of a high frequency snare.

FIG. 7 is an overall summary view of a high frequency forceps.

FIG. 8 is a partial cross-sectional plan view of primary parts of a high frequency forceps.

FIG. 9 is a partial cross-sectional plan view of primary parts of a high frequency forceps.

FIG. 10A is a side view of primary parts of a high frequency forceps.

FIG. 10B is a plan view of primary parts of a high frequency forceps according to Fig. 10A.

## DETAILED DESCRIPTION OF THE INVENTION

A first embodiment of the invention will be explained with reference to FIG.S. 1 and 2.

A high frequency forceps (high frequency treatment instrument) 1 includes an electrical circuit 7 which is connected via a connection cord 3 to a high frequency power source 2 which generates a high frequency voltage in which a pair of forceps pieces (treatment electrodes) 6A and 6B which performs conduction treatment to a target tissue S by using high frequency current between a return electrode 5 with the high frequency voltage applied from the high frequency power source 2, and is known as a monopolar high frequency forceps. The high frequency forceps includes an elongated flexible tube 8 in which the pair of forceps pieces 6A and 6B are arranged at distal end thereof, an operation part 10 for opening and closing the pair of forceps pieces 6A and 6B, and an impedance adjust part 11 that is connected to the electrical circuit 7 and, based on the frequency of the high frequency power source 2, changes at least one of an actual number part and an imaginary number part of the impedance of the electrical circuit 7.

The flexible tube 8 includes a stainless steel coil sheath 12 and a resin outer sheath 13 that covers an outer side of the coil sheath 12 in which operatio, wires 15A and 15B are arranged so as to advance and retreat with respect to the flexible tube 8. A support part 16 is connected to the distal end of the coil sheath 12 to which the pair of forceps pieces 6A and 6B are supported so as to pivot freely. Proximal ends of the pair of forceps pieces 6A and 6B and distal ends of the operation wires 15A and 15B are rotatably connected to the support part 16.

The operation part 10 includes an operation part body 17 to which the proximal end of the flexible tube 8 is connected, and a slider 18 which is disposed so as to advance and retreat with respect to the operation part body 17. In the slider 18, an electrode terminal 20 to which a connection cord 3 is connected is disposed, and proximal ends of the operation wires 15A and 15B are connected to the slider 18 via a wire attachment part 21.

The impedance adjust part 11 includes a capacitor part 22 whose capacitance is set to a predetermined value so as to minimize the impedance of the electrical circuit 7. The capacitance of the capacitor part 22 is based on the value of the impedance when a predetermined high frequency current is supplied to the electrical circuit 7, which is formed when the electrode terminal 20 and the high frequency power source 2 are connected via the connection cord 3 with respect to a high frequency forceps without the impedance adjust part 11 and when the target tissue S is clasped by the pair of forceps pieces 6A and 6B with the return plate 5 disposed thereto. The impedance adjust part 11 is connected in series to the electrical circuit 7. In the embodiment as shown in F. 2, it is connected in series to the electrode terminal 20.

The capacitor part may have a capacitance that is determined based on the impedance of an electrical circuit formed by connecting the electrode terminal 20 and the high frequency power source 2 via the connection cord 3 without clasping the target tissue S. Alternatively, it may have a capacitance that is determined based on an electrical circuit of a high frequency forceps single unit formed from the pair of forceps pieces 6A and 6B, the operation wires 15A and 15B, and the electrode terminal 20.

Subsequently, a function of the high frequency forceps 1 will be described.

Without attaching the impedance adjust part 11, the electrode terminal 20 and the high frequency power source 2 are connected via the connection cord 3, and a material having similar impedance as the target tissue S is mounted on the return plate 5. The slider 18 is then retreated from the operation part body 17 and clasped by the pair of forceps pieces 6A and 6B. In this state, a high frequency current having a predetermined frequency is supplied, and the impedance of the overall electrical circuit 7 is measured.

Equation (1) expresses the magnitude ($|Z_0|\Omega$) when the impedance at this time is $Z_0$. Here ,$R_0$, $L_0$, and $C_0$ respectively represent the resistance ($\Omega$),the inductance (H), and the capacitance (F) of an existing electrical circuit, while f represents the frequency of the high frequency current.

$$|Z_0|= \sqrt{R_0^{\,2} + \left(2\pi f L_0 - \frac{1}{2\pi f C_0}\right)^2} \qquad \cdots (1)$$

Subsequently, based on the magnitude of the measured impedance, when the impedance adjust part 11 is connected in series, a capacitance ($C_1$) which minimizes a portion relating to the square of the imaginary number part of the equation (1) is calculated. An impedance adjust part 11 which includes a capacitor part 22 whose capacitance satisfies this capacitance ($C_1$) is connected in series to the electrode terminal 20. The impedance adjust part 11 can be connected after measuring the impedance at that point, or an impedance adjust part 11 that is designed based on an impedance ascertained beforehand can be assembled in a manufacturing process of the high frequency forceps 1.

An endoscope having an unillustrated treatment instrument channel is inserted into a test body where the return plate 5 is placed, and a distal end part of the endoscope is disposed near the target tissue S. The flexible tube 8 of the high frequency forceps 1 including the impedance adjust part 11 is then inserted into the channel, and is protruded from the channel towards the target tissue S.

The high frequency forceps 1 and the high frequency power source 2 are connected using the connection cord 3, the slider 18 is retreated with respect to the operation part body 17, and the target tissue S is clasped by the pair of forceps pieces 6A and 6B. In this state, a high frequency current having a predetermined frequency is supplied.

The magnitude ($Z_1$) of the impedance of the high frequency forceps 1 is a value expressed by equation (2).

$$|Z_1|= \sqrt{R_0^{\,2} + \left(2\pi f L_0 - \frac{1}{2\pi f C_1}\right)^2} \qquad \cdots (2)$$

In this case, since this minimizes a portion relating to the square of the imaginary number part of the impedance in the state where the impedance adjust part 11 is connected to the electrical circuit 7, a large part of the magnitude of the impedance can be constituted of only the resistance component. The target tissue S clasped by the pair of forceps pieces 6A and 6B is cauterized, and operations such as stopping bleeding and dissection are performed.

According to the high frequency forceps 1, since the impedance adjust part 11 minimizes the imaginary part of the impedance of the electrical circuit, high frequency current can be efficiently supplied and power consumption can be reduced. Depending on the measurement of the impedance, an unillustrated coil part whose inductance is set to a predetermined value can be used instead of the capacitor part 22.

This similarly minimizes the portion of the equation (2) relating to the square of the imaginary number part of the impedance of the electrical circuit, enabling a large part of the magnitude of the impedance to be constituted of only the resistance component.

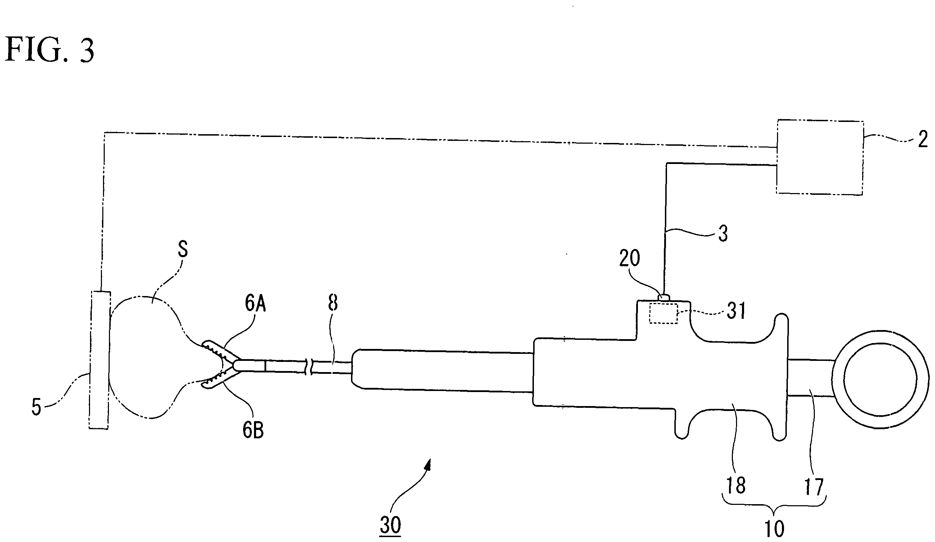

Subsequently, a second embodiment will be explained with reference to FIG. 3.

Constituent components identical with those of the first embodiment are denoted with the same reference numerals, and identical descriptions will be omitted.

The second embodiment differs from the first embodiment in that an impedance adjust part 31 of a high frequency forceps 30 according to the embodiment further includes an unillustrated electrical resistor part.

The electrical resistor part changes the value of the actual number part of the impedance of the electrical circuit. The resistance value of the electrical resistor part is based on the resistance value of the electrical circuit which is formed when the electrode terminal 20 and the high frequency power source 2 are connected via the connection cord 3 to a high frequency forceps without the impedance adjust part 31 and when the target tissue S is clasped by the pair of forceps pieces 6A and 6B with the return plate 5 disposed thereto. For example, when the output of the high frequency power source 2 is below a proximity of rating, the resistance value of the overall electrical circuit 7 when the impedance adjust part 31 is connected is set so as to be increased. The impedance adjust part 31 is connected in series with the electrical circuit in the same manner as the first embodiment.

Subsequently, a function of the high frequency forceps 30 according to the embodiment will be explained.

As in the first embodiment, without attaching the impedance adjust part 31 with a high frequency current having a predetermined frequency being supplied, the impedance of the overall electrical circuit is measured.

At this time, for example, when the output of the high frequency power source 2 falls below a proximity of rating, the resistance value needed to raise the output to the proximity of rating is calculated. Based on the magnitude of the measured impedance, an impedance adjust part 31 whose resistance value is adjusted so as to reflect the actual number part in the equation (1) is connected in series to the electrode terminal 20.

The flexible tube 8 of the high frequency forceps 30 including the impedance adjust part 31 is then inserted into the channel, the target tissue S is clasped by the pair of forceps pieces 6A and 6B, and a high frequency current having a predetermined frequency is supplied. Since the magnitude of the impedance when the impedance adjust part 31 is connected to the electrical circuit 7 is the resistance value at the proximity of rating output, the target tissue S clasped by the pair of forceps pieces 6A and 6B can be cauterized, and operations such as stopping bleeding and dissection can be performed, in a desired state.

According to the high frequency forceps 30, even if the impedance when the high frequency forceps 30 is connected is too low with respect to the load characteristics of the high frequency power source 2, sufficient power in respect of the load characteristics of the high frequency power source can be output.

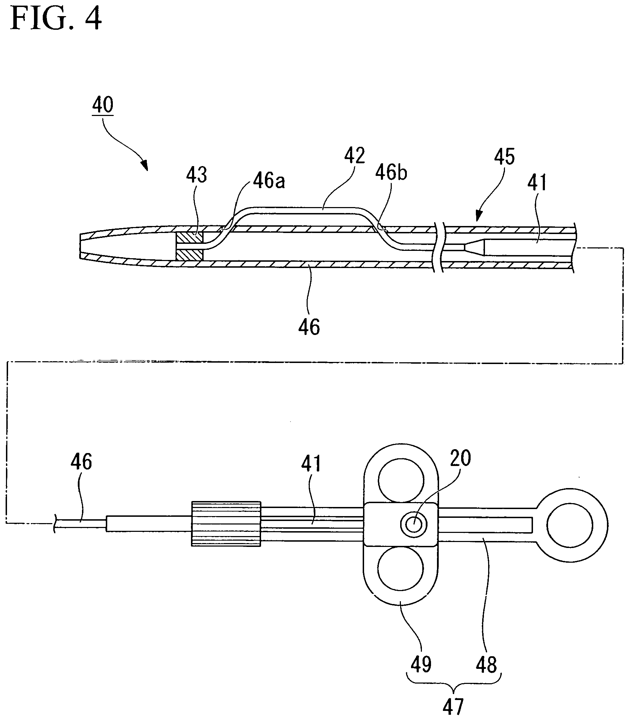

Subsequently, a third embodiment will be explained with reference to FIG. 4.

Constituent components identical with those of the other embodiment described above are denoted with the same reference numerals, and identical descriptions will be omitted.

The third embodiment differs from the first embodiment in that a high frequency treatment instrument according to the embodiment is a papillotomy knife 40.

A knife (treatment electrode) 42 is connected to a distal end of an operation wire 41. A distal end of the knife 42 is connected to a distal end securing part 43 for securing the distal end of the knife 42. A flexible tube 45 includes a resin sheath 46 in which holes 46a and 46b for exposing the knife 42 to the outside are provided in the distal end thereof.

An operation part 47 includes an operation part body 48 to which a proximal end of the sheath 46 is connected, and a slider 49 which can advance and retreat with respect to the operation part body 48. The slider 49 is provided with an electrode terminal 20 to which an unillustrated impedance adjust part, similar to the impedance adjust part 11 of the first embodiment, is connected in series. As in the first embodiment, the electrode terminal 20 and the operation wire 41 are connected.

According to the papillotomy knife 40, since an impedance adjust part similar to the impedance adjust part 11 of the first embodiment is provided, when a high frequency current at a predetermined frequency is supplied, similar effects to those of the high frequency forceps 1 according to the first embodiment can be obtained.

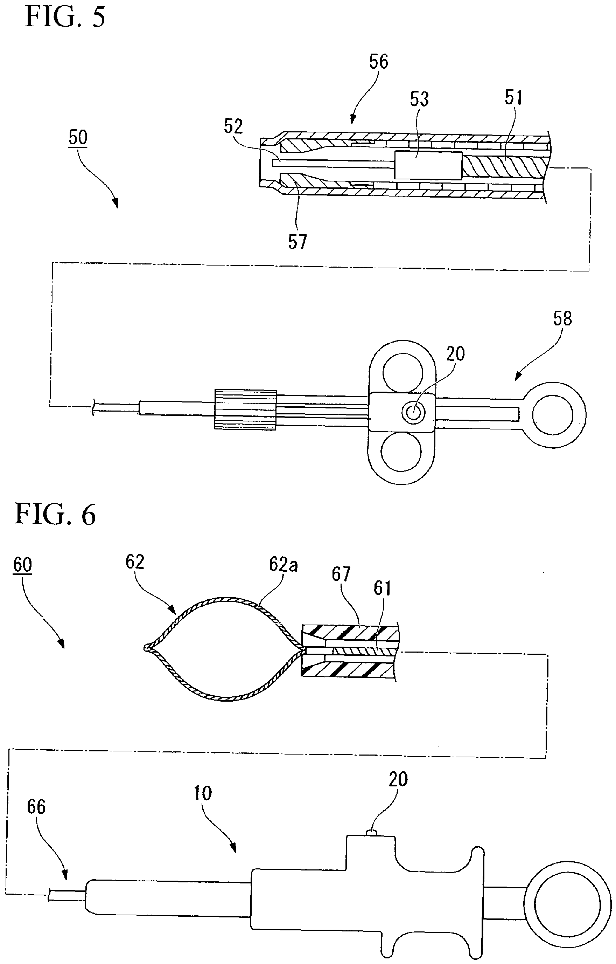

Subsequently, a fourth embodiment will be explained with reference to FIG. 5.

Constituent components identical with those of the other embodiment described above are denoted with the same reference numerals, and identical descriptions will be omitted.

The fourth embodiment differs from the third embodiment in that a high frequency treatment instrument according to this embodiment is a high frequency knife 50.

A knife for electrode (treatment electrode) 52 is connected to a distal end of an operation wire 51 via a stopper support part 53. A stopper member 57 is provided at a distal end of a flexible tube 56 in order to regulate the knife for electrode so that it does not protrude more than a predetermined length. An operation part 58 is provided with an electrode terminal 20 to which an unillustrated impedance adjust part, similar to the impedance adjust part 11 of the first embodiment, is connected in series.

According to the high frequency knife 52, since an impedance adjust part similar to the impedance adjust part 11 of the first embodiment is provided, when a high frequency current at a predetermined frequency is supplied, similar effects to those of the high frequency forceps 1 can be obtained.

Subsequently, a fifth embodiment will be explained with reference to FIG. 6.

Constituent components identical with those of the other embodiment described above are denoted with the same reference numerals, and identical descriptions will be omitted.

The fifth embodiment differs from the first embodiment in that a high frequency treatment instrument according to this embodiment is a high frequency snare 60.

A snare loop (treatement electrode) 62, which is formed of a looped eastic wire 62a, is connected to a distal end of an operation wire 61.

A flexible tube 66 is provided with only a sheath 67, which is different from the first embodiment, and an operation wire is disposed inside the sheath 67 so as to advance and retreat. An operation part 10 is provided with an electrode terminal 20 to which an unillustrated impedance adjust part, similar to the impedance adjust part 11 of the first embodiment, is connected in series.

According to the high frequency snare 60, since an impedance adjust part similar to the impedance adjust part 11 of the first embodiment is provided, when a high frequency current at a predetermined frequency is supplied, similar effects to those of the high frequency forceps 1 according to the first embodiment can be obtained.

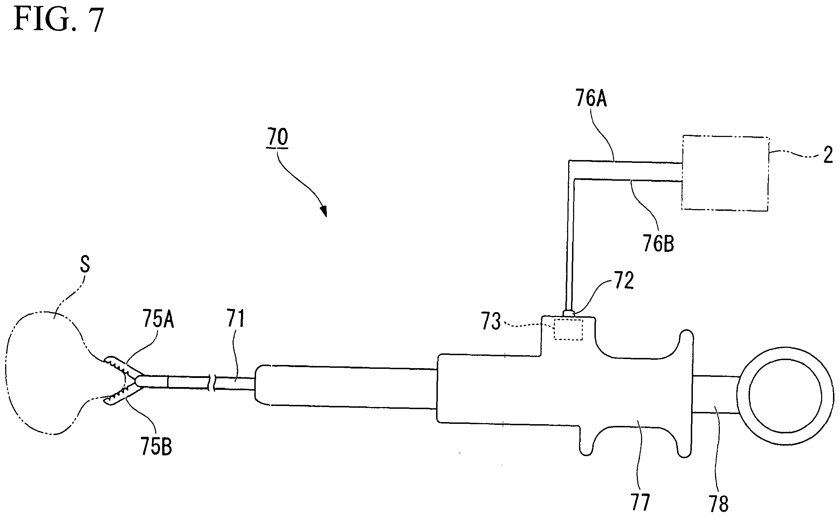

Subsequently, a sixth embodiment will be explained with reference to FIG. 7.

Constituent components identical with those of the other embodiment described above are denoted with the same reference numerals, and identical descriptions will be omitted.

The sixth embodiment differs from the first embodiment in that a high frequency forceps (treatment instrument) 70 according to this embodiment is a bipolar type.

Unillustrated operation wires are arranged inside a flexible tube 71 in mutually insulated state, and connected to an electrode terminal 72 in the insulated state. The electrode terminal 72 is constituted separately for positive pole and negative pole. As in the first embodiment, an impedance adjust part 73 is connected in series to the electrode terminal 72. A first forceps piece (first electrode) 75A and a second forceps piece (second electrode) 75B, which generate mutually different potential differences, are respectively connected to distal ends of the operation wires. Accordingly, connection cords 76A and 76B are separately provided for the first forceps piece 75A and the second forceps piece 75B respectively.

Subsequently, a function of the high frequency forceps 70 according to the embodiment will be explained.

First, without attaching the impedance adjust part 73, the electrode terminal 72 and the high frequency power source 2 are connected via the connection cords 76A and 76B, the slider 77 is retreated with respect to the operation part body 78, and a material having the same impedance as the target tissue S is clasped by the first forceps piece 75A and the second forceps piece 75B. Without attaching the impedance adjust part 73, a high frequency current at a predetermined high frequency is supplied and the impedance of the overall electrical circuit is measured.

As in the first embodiment, based on the magnitude of the measured impedance, the impedance adjust part 73 including an unillustrated capacitor part having a capacitance which is equal to the capacitance (C1) in the equation (2) is connected in series to the electrode terminal 20.

The flexible tube 71 of the high frequency forceps 70 including the impedance adjust part 73 is then inserted into the channel, the target tissue S is clasped by the first forceps piece 75A and the second forceps piece 75B, and a high frequency current having a predetermined frequency is supplied. Since this minimizes the portion relating to the square of the imaginary number part of the impedance in the state where the impedance adjust part 73 is connected to the electrical circuit, a large part of the magnitude of the impedance becomes only the resistance component. Thus the target tissue S clasped by the first forceps piece 75A and the second forceps piece 75B is cauterized, and operations such as stopping bleeding and dissection are performed.

According to the high frequency forceps 70, similar effects to those of the first embodiment can be obtained.

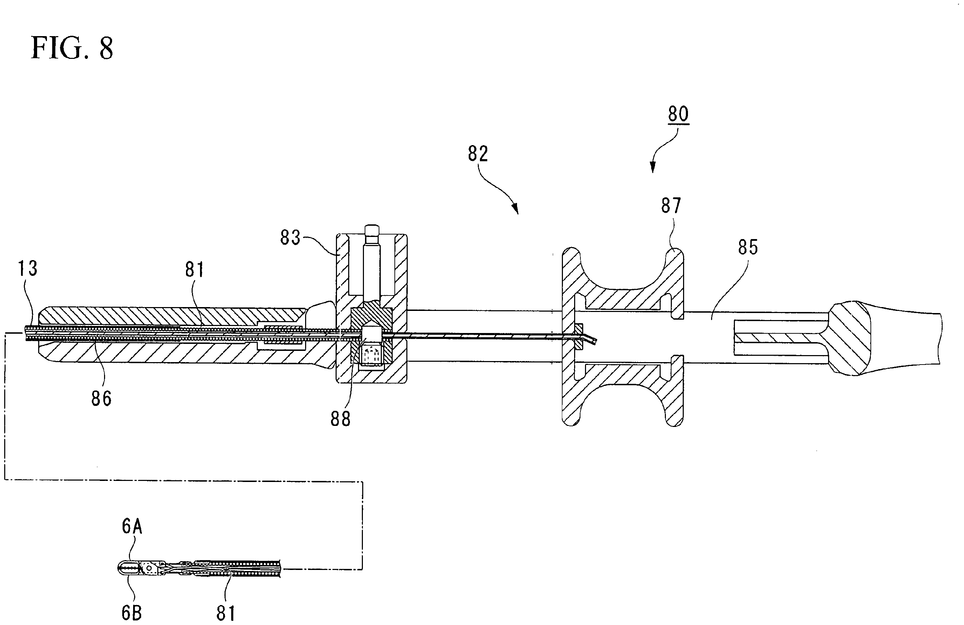

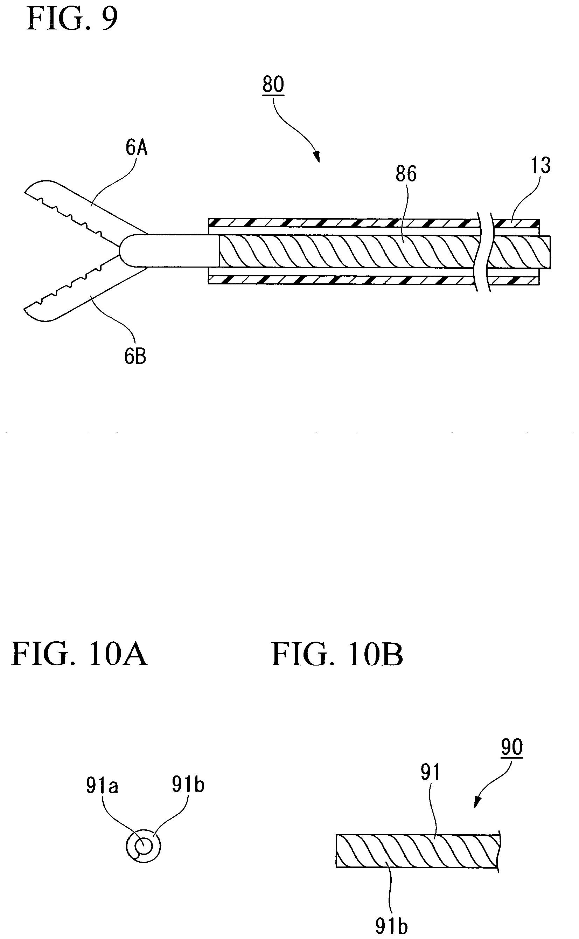

Subsequently, a seventh embodiment will be explained with reference to FIGS. 8 and 9.

Constituent components identical with those of the other embodiment described above are denoted with the same reference numerals, and identical descriptions will be omitted.

The seventh embodiment differs from the first embodiment in that a coil sheath 81 of a high frequency forceps 80 according to the embodiment is an impedance adjust part.

The coil sheath 81 is formed by winding wire formed of a material with a low resistance value such as tungsten or molybdenum. The diameter and winding number of the coil sheath 81 is adjusted such that, when a high frequency current having a predetermined frequency is supplied, such an inductance that a portion relating to the square of the imaginary number part of the impedance of the electrical circuit becomes minimized is provided.

An operation part 82 includes an operation part body 85 including a connection part 83 in which the electrode terminal 20 is provided, and a slider 87 to which a proximal end of an operation wire 86 is connected. The connection part 83 is provided with a coil attachment part 88 for connecting the proximal end of the coil sheath 81 to the electrode terminal 20.

Subsequently, a function of the high frequency forceps 80 according to the embodiment will be explained.

First, the electrode terminal 20 and the high frequency power source 2 are connected via the connection cord 3, and a material having similar impedance as the target tissue S is mounted on the return plate 5. The slider 87 is then retreated with respect to the operation part body 85 and clasped by the pair of forceps pieces 6A and 6B. In this state, a high frequency current having a predetermined frequency is supplied, and the impedance of the overall electrical circuit is measured.

Based on the magnitude of the measured impedance, an inductance of the electrical circuit that minimizes the portion relating to the square of the imaginary number part of the equation (1) is calculated. The diameter and winding number of the coil sheath 81 are respectively changed to predetermined values to satisfy the inductance. The diameter and winding number of the coil sheath 81 can respectively be changed to predetermined values after measuring the impedance at that point, or a coil sheath 81 that is designed based on an impedance ascertained beforehand can be assembled in a manufacturing process of the high frequency forceps 80.

Thereafter, a high frequency current having a predetermined frequency is supplied by an operation similar to that of the first embodiment.

According to the high frequency forceps 80, the coil sheath 81 can be used as an impedance adjust part for adjusting the inductance by changing the diameter, material, and winding number of the coil sheath 81. Therefore, the high frequency forceps structure can be simplified without adding new members.

Subsequently, an eighth embodiment will be explained with reference to FIG. 10.

Constituent components identical with those of the other embodiment described above are denoted with the same reference numerals, and identical descriptions will be omitted.

The eighth embodiment differs from the first embodiment in that an operation wire 91 of a high frequency forceps 90 according to the embodiment includes an insulating core wire member 91a, and a side wire member 91b that is wound around the core wire member 91a, the operation wire 91 functioning as impedance adjust part.

The side wire member 91b is formed of a material with a low resistance value such as tungsten or molybdenum. The diameter and winding number of the side wire member 91b and the diameter of the core member 91a are adjusted such that, when a high frequency current having a predetermined frequency is supplied to the operation wire 91, such an inductance that the portion relating to the square of the imaginary number part of the impedance of the electrical circuit becomes minimized is provided.

Subsequently, a function of the high frequency forceps 90 according to the embodiment will be explained.

First, as in the first embodiment, a high frequency current having a predetermined frequency is supplied and the impedance of the overall electrical circuit is measured.

Based on the magnitude of the measured impedance, an inductance that minimizes the portion relating to the square of the imaginary number part of the equation (1) is calculated. The diameter and winding number of the side wire member 91b and the diameter of the core member 91a are adjusted so as to satisfy the inductance.

Thereafter, a high frequency current having a predetermined frequency is supplied by an operation similar to that of the first embodiment.

According to the high frequency forceps 90, the operation wire 91 functions as an impedance adjust part which is able to adjust the iuductance by changing the diameter, material quality, and winding number of the side wire member 91b of the operation wire 91.

Therefore, the high frequency forceps structure can be simplified without adding new members.

The technical field of the present invention is not limited to the embodiments described and illustrated above; it is possible to make various modifications without departing from the scope of the present invention.

For example, in the first embodiment, the impedance adjust part 11 is connected in series with the electrode terminal 20 because it has a specific configuration for each treatment instrument, however, there is no limitation on this, and it can, for example, be connected in series to a connector portion of a general-purpose connection cord.

Also, depending on the impedance measurement result, the impedance adjust part can include a coil part, a capacitor part, and an electrical resistor part, each of which is independently provided; alternatively, it can be configured by a combination of at least two of these.

Moreover, the capacitance, inductance, and resistance of the impedance adjust part can be made changeable even after the impedance adjust part is connected.

**Claims**

1. A high frequency treatment instrument (1, 30, 40, 50, 60, 70, 80, 90) comprising

an electrical circuit (7) which is connectable to a high frequency power source (2) being configured to generate a high frequency voltage in which at least one treatment electrode (6A, 6B, 42, 52, 62) is included for performing conduction treatment using a high frequency current to a target tissue (S) by applying a high frequency voltage from the high frequency power source (2);

an impedance adjustment part (11, 31, 73) which includes a coil part having an inductance, a capacitor having a capacitance, or an electrical resistor connectable to the electrical circuit (7) in series, and based on the frequency of the high frequency power source (2) is configured to change at least one of a real number part and an imaginary number part of an impedance of the electrical circuit (7); and

an operation part body (17),

**characterized by**

a slider (18) disposed so as to advance and retreat with respect to the operation part body (17), the slider comprising an electrode terminal (20, 72), wherein the impedance adjustment part (11, 31, 73) is configured to be connectable to electrode terminal (20, 72), and the electrical circuit (7) is configured to supply the high frequency voltage to the at least one treatment electrode (6A, 6B, 42, 52, 62) via the electrode terminal (20, 72) when the impedance adjustment part (11, 31, 73) is connected to the electrode terminal (20, 72) in series and without the impedance adjustment part (11, 31, 73) being connected to the electrode terminal (20, 72).

2. The high frequency treatment instrument (1, 30, 40, 50, 60, 70, 80, 90) according to claim 1, wherein the treatment electrode (6A, 6B, 42, 52, 62) includes a first electrode (75A) and a second electrode (75B) which are configured to create mutually different potential differences.

**Patentansprüche**

1. Hochfrequenzbehandlungsgerät (1, 30, 40, 50, 60, 70, 80, 90), umfassend
eine elektrische Schaltung (7), die an eine Hochfrequenzenergiequelle (2) anschließbar ist, die konfiguriert ist, um eine Hochfrequenzspannung zu erzeugen, in der mindestens eine Behandlungselektrode (6A, 6B, 42, 52, 62) enthalten ist, um eine Leitungsbehandlung unter Verwendung eines Hochfrequenzstroms an einem Zielgewebe (S) durch Anlegen einer Hochfrequenzspannung aus der Hochfrequenzenergiequelle (2) auszuführen;
ein Impedanzanpassungsteil (11, 31, 73), das ein Spulenteil, das eine Induktivität aufweist, einen Kondensator, der eine Kapazität aufweist, oder einen elektrischen Widerstand, der mit der elektrischen Schaltung (7) in Reihe geschaltet werden kann, umfasst und basierend auf der Frequenz der Hochfrequenzenergiequelle (2) konfiguriert ist, um mindestens einen von einem reellen Zahlenanteil und einem imaginären Zahlenanteil einer Impedanz der elektrischen Schaltung (7) zu ändern; und
einen Bedienungsteilkörper (17),
**gekennzeichnet durch**
einen Schieber (18), der angeordnet ist, um sich hinsichtlich dem Bedienungsteilkörper (17) vorwärts und rückwärts zu bewegen, wobei der Schieber eine Elektrodenklemme (20, 72) umfasst, wobei das Impedanzanpassungsteil (11, 31, 73) konfiguriert ist, um an die Elektrodenklemme (20, 72) anschließbar zu sein, und die elektrische Schaltung (7) konfiguriert ist, um die Hochfrequenzspannung der mindestens einen Behandlungselektrode (6A, 6B, 42, 52, 62) über die Elektrodenklemme (20, 72) zuzuführen, wenn das Impedanzanpassungsteil (11, 31, 73) mit der Elektrodenklemme (20, 72) in Reihe geschaltet ist, und ohne dass das Impedanzanpassungsteil (11, 31, 73) an die Elektrodenklemme (20, 72) angeschlossen ist.

2. Hochfrequenzbehandlungsgerät (1, 30, 40, 50, 60, 70, 80, 90) nach Anspruch 1, wobei die Behandlungselektrode (6A, 6B, 42, 52, 62) eine erste Elektrode (75A) und eine zweite Elektrode (75B) umfasst, die konfiguriert sind, um voneinander unterschiedliche Potentialdifferenzen zu erzeugen.

**Revendications**

1. Instrument de traitement haute fréquence (1, 30, 40, 50, 60, 70, 80, 90) comprenant
un circuit électrique (7) qui peut être connecté à une source d'alimentation haute fréquence (2) qui est configurée pour générer une tension haute fréquence dans lequel au moins une électrode de traitement (6A, 6B, 42, 52, 62) est incluse pour réaliser un traitement par conduction à l'aide d'un courant haute fréquence sur un tissu cible (S) par application d'une tension haute fréquence provenant de la source d'alimentation haute fréquence (2) ;
une partie d'ajustement d'impédance (11, 31, 73) qui comprend une partie de bobine ayant une inductance, un condensateur ayant une capacité, ou une résistance électrique pouvant être connectée au circuit électrique (7) en série, et, sur la base de la fréquence de la source d'alimentation haute fréquence (2), est configurée pour changer au moins l'une d'une partie de nombre réel et d'une partie de nombre imaginaire d'une impédance du circuit électrique (7) ; et
un corps de partie opérationnelle (17),
**caractérisé par**
un coulisseau (18) disposé de façon à avancer et à reculer par rapport au corps de partie opérationnelle (17), le coulisseau comprenant une borne d'électrode (20, 72),
la partie d'ajustement d'impédance (11, 31, 73) étant configurée pour pouvoir être connectée à la borne d'électrode (20, 72), et le circuit électrique (7) étant configuré pour fournir la tension haute fréquence à l'au moins une électrode de traitement (6A, 6B, 42, 52, 62) par l'intermédiaire de la borne d'électrode (20, 72) lorsque la partie d'ajustement d'impédance (11, 31, 73) est connectée à la borne d'électrode (20, 72) en série et sans que la partie d'ajustement d'impédance (11, 31, 73) ne soit connectée à la borne d'électrode (20, 72).

2. Instrument de traitement haute fréquence (1, 30, 40, 50, 60, 70, 80, 90) selon la revendication 1, dans lequel l'électrode de traitement (6A, 6B, 42, 52, 62) comprend une première électrode (75A) et une seconde électrode (75B) qui sont configurées pour créer des différences de potentiel mutuellement différentes.

FIG. 1

EP 1 867 296 B1

FIG. 2

FIG. 3

EP 1 867 296 B1

# FIG. 4

## FIG. 5

## FIG. 6

# FIG. 7

FIG. 8

EP 1 867 296 B1

# FIG. 9

# FIG. 10A

# FIG. 10B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005058344 A
- EP 1743589 A
- WO 0115617 A